# EUROPÄISCHE PATENTSCHRIFT

(11) **EP 3 099 785 B1**
(45) Veröffentlichungstag und Bekanntmachung des Hinweises auf die Patenterteilung: **31.07.2019**
(21) Anmeldenummer: 15703510.6
(22) Anmeldetag: 28.01.2015
(51) Int. Cl.: C12N 11/14, A61K 41/00, A61B 17/425, C12N 5/071, C12N 5/076

(54) **VERFAHREN ZUR HERSTELLUNG DER BEWEGLICHKEIT VON IMMOBILEN ZELLEN**
METHOD FOR MOBILIZING IMMOBILIZED CELLS
PROCÉDÉ DE RÉTABLISSEMENT DE LA MOBILITÉ DE CELLULES IMMOBILES

(30) Priorität: 31.01.2014 DE 102014201760
(43) Veröffentlichungstag der Anmeldung: 07.12.2016
(73) Patentinhaber: Leibniz-Institut für Festkörper- und Werkstoffforschung Dresden e.V., 01069 Dresden (DE)
(72) Erfinder: SCHMIDT, Oliver G., 01219 Dresden (DE)
(74) Vertreter: Rauschenbach, Marion
(86) Internationale Anmeldenummer: PCT/EP2015/051650
(87) Internationale Veröffentlichungsnummer: WO 2015/113984

(56) Entgegenhaltungen:
- DE-A1-102012 212 427
- US-A1- 2003 204 128
- VERONIKA MAGDANZ ET AL: "Development of a Sperm-Flagella Driven Micro-Bio-Robot", ADVANCED MATERIALS, Bd. 25, Nr. 45, 1. Dezember 2013 (2013-12-01), Seiten 6581-6588, XP055172822, ISSN: 0935-9648, DOI: 10.1002/adma.201302544
- SHIRLY BEN-DAVID MAKHLUF ET AL: "Loading Magnetic Nanoparticles into Sperm Cells Does Not Affect Their Functionality", LANGMUIR, Bd. 22, Nr. 23, 1. November 2006 (2006-11-01), Seiten 9480-9482, XP055172743, ISSN: 0743-7463, DOI: 10.1021/la061988z
- Peyer et al: "Magnetic Helical Micromachines", , 21. März 2013 (2013-03-21), XP002736650, Gefunden im Internet: URL:http://www.msrlpublications.ch/btw/fil es/Chemistry_A_Peyer_2012.pdf [gefunden am 2015-03-02]

## Beschreibung

Die Erfindung bezieht sich auf die Gebiete der Mikrotechnologien, der Biologie und der Medizin und betrifft ein Verfahren zur Herstellung der Beweglichkeit von immobilen Zellen, wie es beispielsweise bei der in-vivo- oder in-vitro-Fertilisation angewandt werden kann.

Es sind verschiedene Verfahren bekannt, im Falle von geringer Mobilität von Zellen, beispielsweise Spermien, die Fertilisation sowohl im Körper als auch außerhalb, zu erreichen und/oder ihre Erfolgsrate zu verbessern.

Es ist ein Verfahren bekannt, bei dem eine etwa 200 µm große Eizelle oder ein größerer Embryo mit einer Schicht aus magnetischen Partikeln versehen wird und durch Anlegen eines magnetischen Feldes über einen Permanentmagneten oder einen Elektromagneten die Eizelle oder der Embryo in die Gebärmutter transportiert und dort stabilisiert wird (US 6,695,766 B4). Dazu werden magnetische Partikel auf ihrer Oberfläche mit reaktiven Gruppen versehen. Die magnetischen Partikel können einen Durchmesser von 0,1µm bis 200 µm aufweisen. Diese magnetischen Partikel werden mit der Eizelle oder dem Embryo zusammengebracht, so dass die reaktiven Gruppen der magnetischen Partikel mit den reaktiven Gruppen auf der Oberfläche der Eizelle oder des Embryo's reagieren können.

Weiterhin ist nach der DE 10 2012 212 427 A1 ein Verfahren zur kontrollierten Bewegung von motilen Zellen in flüssigen oder gasförmigen Medien bekannt, bei dem motile Zellen in oder an ein magnetisches Partikel ein- oder angebracht werden und durch Anlegen eines externen Magnetfeldes die magnetischen Partikel mit den motilen Zellen gerichtet bewegt werden.

Weiterhin sind Verfahren bekannt, mit denen künstliche Helix- und flexible Flagellastrukturen in Mikrometergröße hergestellt werden können, die über ein externes Magnetfeld in Flüssigkeiten bewegt und gelenkt werden können (R. Dreyfus, J. Baudry, M. L. Roper, M. Fermigier, H. A. Stone and J. Bibette, Nature, 2005, 437, 862-865; L. Zhang, J. J. Abbott, L. Dong, B. E. Kratochvil, D. Bell and B. J. Nelson, Appl. Phys. Lett., 2009, 94, 064107).

Nachteilig bei den bekannten Verfahren ist, dass sie es nicht erlauben, einzelne immobile mikrometergroße Zellen im Körper eines Säugetiers oder eines Menschen mittels eines Magnetfelds zu bewegen und zu steuern. Des Weiteren verbleiben bei den genannten Verfahren die magnetische Partikel stets im Körper eines Säugetiers oder Menschen.

Aufgabe der vorliegenden Erfindung ist es, ein in vitro Verfahren zur Herstellung der Beweglichkeit von einzelnen immobilen Zellen anzugeben, mit dem die Aktivität und gesteuerte Mobilität von zuvor immobilen Zellen realisiert wird.

Die Aufgabe wird durch die in den Ansprüchen angegebene Erfindung gelöst. Vorteilhafte Ausgestaltungen sind Gegenstand der Unteransprüche.

Bei dem erfindungsgemäßen in vitro Verfahren zur Herstellung der Beweglichkeit von immobilen Zellen wird eine immobile Zelle mit einer Mikrostruktur verbunden, wobei die Mikrostruktur mindestens teilweise aus magnetischen Materialien besteht und mittels eines sich zeitlich verändernden dreidimensionalen externen Magnetfeldes eine nichtreziproke Bewegung der Mikrostruktur mit der immobilen Zelle ausgeführt wird.

Vorteilhafterweise werden als immobile Zellen immobile unbewegliche Spermazellen eingesetzt.

Ebenfalls vorteilhafterweise wird eine Mikrostruktur aus einem magnetischen Material oder aus einem Material mit magnetischen Partikeln eingesetzt.

Weiterhin vorteilhafterweise wird als magnetisches Material ferromagnetisches oder paramagnetisches Material eingesetzt.

Vorteilhaft ist es auch, wenn als magnetisches Material Eisen, Eisenoxid, Kobalt oder Nickel oder Legierungen dieser Materialien oder in Verbindung mit anderen Materialien eingesetzt werden, und nichtmagnetische Materialien mit magnetischen Materialien beschichtet eingesetzt werden.

Ebenfalls vorteilhaft ist es, wenn eine Mikrostruktur aus einem Polymer eingesetzt wird, welches magnetische Partikel enthält oder welches mit magnetischen Partikeln oder Materialien ganz oder teilweise beschichtet ist.

Und auch vorteilhaft ist es, wenn eine Mikrostruktur eingesetzt wird, die die Form einer Helixstruktur oder einer künstlichen flexiblen Geißel aufweist.

Weiterhin vorteilhaft ist es, wenn die formschlüssige Verbindung der Mikrostruktur mit der immobilen Zelle durch die Form der Mikrostruktur realisiert wird.

Von Vorteil ist es weiterhin, wenn die Verbindung der Mikrostruktur mit der immobilen Zelle durch biochemische Funktionalisierung von Oberflächen realisiert wird.

Und auch von Vorteil ist es, wenn die Mikrostruktur mit Längen von 1 - 200 µm und Durchmessern von 1 µm bis 20 µm eingesetzt werden.

Und weiterhin von Vorteil ist es, wenn ein sich zeitlich veränderndes dreidimensionales Magnetfeld durch Permanentmagneten oder Elektromagneten realisiert wird.

Die hergestellten beweglichen Zellen eignen sich um im Körper eines Säugetieres oder Menschen verwendet zu werden.

Mit dem erfindungsgemäßen Verfahren zur Herstellung der Beweglichkeit von einzelnen immobilen Zellen kann erstmals die Aktivität und gesteuerte Mobilität von zuvor immobilen Zellen realisiert werden.

Erreicht wird dies, indem die immobile Zelle mit einer Mikrostruktur, die für den Antrieb sorgt, verbunden wird. Die Verbindung erfolgt entweder formschlüssig durch eine mechanische Ankopplung der Mikrostruktur an die Zelle oder aber durch eine biochemische Ankopplung mittels funktionalisierter Oberflächen der Zelle und der jeweiligen Mikrostruktur. Vorteilhafterweise weist die Mikrostruktur bei einer formschlüssigen Verbindung eine ringähnliche Öffnung auf, deren Abmessung an die Größe der immobilen Zelle angepasst oder nur geringfügig kleiner oder größer ausgebildet ist. Die Abmessungen der immobilen Zellen und der Mikrostrukturen liegen dabei im Mikrometerbereich. Die Mikrostrukturen weisen vorteilhafterweise Längen von 1 - 200 µm auf und entsprechen im Durchmesser etwa der Größe einer einzelnen Zelle. Die Verbindung zwischen immobiler Zelle und Mikrostruktur erlaubt einerseits die Bewegung der immobilen Zelle gemeinsam mit der Mikrostruktur und gewährleistet andererseits auch eine lösbare Verbindung, so dass die Mikrostruktur nach Transport der immobilen Zelle an den gewünschten Ort falls erforderlich auch gelöst und von dort gerichtet wieder entfernt werden kann.

Die gerichtete Bewegung der immobilen Zellen mit der Mikrostruktur wird einerseits realisiert durch den Einsatz von magnetischen Materialien aus denen die Mikrostruktur ganz oder teilweise besteht und andererseits durch das Anlegen eines zeitlich variierenden dreidimensionalen externen Magnetfeldes, so dass die Mikrostruktur zusammen mit der Zelle eine nichtreziproke oder zeitlich irreversible Bewegung ("nonreciprocal" or "non-time-reversible-movement") durchführt.

Als magnetische Materialien können vorteilhafterweise Eisen, Eisenoxid, Kobalt oder Nickel oder Legierungen dieser Materialien oder in Verbindung mit anderen Materialien eingesetzt werden. Im Falle, dass die hergestellte Mikrostruktur selbst nicht oder nicht ausreichend magnetische Eigenschaften aufweist, ist sie mit magnetischen Materialien ganz oder teilweise umhüllt.

Eine teilweise oder komplette Umhüllung der Mikrostruktur mit einer biokompatiblen Schutzschicht ist ebenfalls möglich.

Es können auch vorteilhafterweise Polymermaterialien als Mikrostrukturmaterial eingesetzt werden, wobei die Polymere magnetische Materialien enthalten können oder mit diesen ganz oder teilweise beschichtet sind.

Durch die erfindungsgemäße Lösung ist es erstmals möglich, immobile Zellen bewegbar zu machen und ihre Bewegung in eine gewünschte Richtung zu lenken.

Unter immobilen Zellen sollen im Rahmen dieser Erfindung lebende Zellen verstanden werden, die nicht die Fähigkeit aufweisen, sich in flüssigen Medien selbstständig zu bewegen.

Nachdem die immobile Zelle mit der Mikrostruktur verbunden ist, wird ein zeitlich veränderliches externes magnetisches Feld angelegt. Über die Änderung dieses Magnetfeldes wird ein Drehmoment an der besonders geformten Mikrostruktur und dadurch eine nichtreziproke Bewegung der Zelle-Mikrostruktur erzeugt, was zu einer Vorwärtsbewegung führt. Die Bewegungsrichtung der Mikrostruktur mit der Zelle kann ebenfalls mit einem externen Magnetfeld kontrolliert werden. Ein derartiges zeitlich veränderliches dreidimensionales externes magnetisches Feld kann durch einen bewegten Permanentmagneten oder bevorzugt durch eine dreidimensionale Anordnung von Elektromagneten erzeugt werden.

Von besonderer Bedeutung ist die Form der Mikrostruktur. Dabei wird als Form vorteilhafterweise eine Helixstruktur oder eine künstliche flexible Geißel eingesetzt. Durch ein zeitlich veränderliches externes Magnetfeld wird die Helixstruktur in eine Drehbewegung versetzt und bewegt sich dabei in eine Richtung vorwärts. Der Durchmesser der Helixstruktur ist vorteilhafterweise angepasst an den Durchmesser der Zelle. Die Mikrostruktur kann aber auch eine Geißelstruktur sein, die an die immobile Zelle befestigt wird. Die Geißel wird durch ein sich zeitlich veränderliches dreidimensionales externes Magnetfeld in eine schlängelnde Bewegung versetzt, wodurch eine Bewegung der immobilen Zelle realisiert wird.

Durch die Anbringung der Mikrostruktur an eine immobile Zelle wird erfindungsgemäß im Falle einer immobilen Spermazelle quasi eine künstliche Geißel zur Bewegung realisiert.

Das erfindungsgemäße Verfahren könnte zur Unterstützung der natürlichen in-vivo Fertilisation angewandt werden, wenn Spermien eine stark verringerte oder gar keine Mobilität mehr aufweisen. Dadurch würde eine erhöhte Erfolgsrate bei Befruchtungen erreicht und es würde eine alternative Reproduktionstechnik eröffnet, da eine Entnahme der Eizelle aus dem Tierkörper oder dem menschlichen Körper nicht notwendig wäre. Die mit der Mikrostruktur verbundenen Spermazellen könnten direkt in die Gebärmutter eingebracht werden. Durch die Anwendung eines sich zeitlich ändernden Magnetfeldes würde die Spermazelle damit gezielt durch den Eileiter zur Eizelle bewegt, und es käme zur Befruchtung.

Durch die spezielle Anbindung der Mikrostruktur kann diese auch von der immobilen Zelle entfernt werden, beispielsweise bei einer Helixstruktur durch Umkehr der Rotationsrichtung des Magnetfeldes und damit auch Umkehr der Rotation der Mikrostruktur. Damit könnte die Mikrostruktur aus dem Tierkörper oder menschlichen Körper ganz entfernt werden.

Das erfindungsgemäße Verfahren wäre aber auch einsetzbar für die in-vitro Fertilisation mit dem Transport der Zellen zur Eizelle außerhalb des Körpers.

Der Vorteil der erfindungsgemäßen Lösung würde darin bestehen, dass ursprünglich nicht bewegliche Zellen im Tierkörper oder menschlichen Körper bewegt werden könnten und dass die magnetischen Materialien nicht im Körper verbleiben müssten und daher keine negativen Auswirkungen auf den Organismus hätten.

Nachfolgend wird die Erfindung an einem Ausführungsbeispiel näher erläutert.

### Referenz Beispiel

Eine biokompatible polymere Helixstruktur aus ORMOCOMP® (micro resist technology GmbH) wird durch drei-dimensionales Laserschreiben hergestellt. Die Helixstruktur hat einen Durchmesser, der etwas kleiner ist als die Breite eines menschlichen Spermiumkopfes, also etwa 3 µm, und eine Länge von 80 µm. Die Helixstruktur wird dann mit einer dünnen Eisenschicht von 30 nm Dicke mittels Aufdampfverfahren beschichtet. An einem Ende ist die Helixstruktur ringförmig gestaltet. Die ferromagnetische Helixstruktur wird durch ein zeitlich veränderliches externes Magnetfeld bewegt und an ein unbewegliches Spermium mechanisch angekoppelt. Dies passiert außerhalb des menschlichen Körpers, also in-vitro. Nach der mechanischen Ankopplung befindet sich die unbewegliche natürliche Spermiengeißel inmitten der Helixstruktur. Ein oder mehrere an Helixstrukturen gekoppelte Spermien werden anschließend per Insemination in die Gebärmutter des weiblichen Körpers injiziert. Anschließend werden die an Helixstrukturen gekoppelten Spermien durch ein rotierendes externes Magnetfeld in Bewegung gesetzt, durch den Eileiter gelenkt und schließlich mit der Eizelle in Kontakt gebracht, wo es zur erfolgreichen Befruchtung kommt. Danach wird die Rotationsrichtung des magnetischen Feldes umgedreht, was dazu führt, dass sich die Helixstruktur in die entgegengesetzte Richtung bewegt und sich daher von dem Spermium trennt und abkoppelt. Abschließend wird die Helixstruktur aus dem Körper der Frau hinausbefördert.

## Patentansprüche

1. In vitro Verfahren zur Herstellung der Beweglichkeit von immobilen Zellen, bei dem eine immobile Zelle mit einer Mikrostruktur verbunden wird, wobei die Mikrostruktur mindestens teilweise aus magnetischen Materialien besteht und mittels eines sich zeitlich verändernden dreidimensionalen externen Magnetfeldes eine nichtreziproke Bewegung der Mikrostruktur mit der immobilen Zelle ausgeführt wird, wobei als immobile Zellen immobile unbewegliche Spermazellen eingesetzt werden, und wobei eine Mikrostruktur eingesetzt wird, die die Form einer Helixstruktur oder einer künstlichen flexiblen Geißel aufweist.

2. Verfahren nach Anspruch 1, bei dem eine Mikrostruktur aus einem magnetischen Material oder aus einem Material mit magnetischen Partikeln eingesetzt wird.

3. Verfahren nach Anspruch 1, bei dem als magnetisches Material ferromagnetisches oder paramagnetisches Material eingesetzt wird.

4. Verfahren nach Anspruch 1, bei dem als magnetisches Material Eisen, Eisenoxid, Kobalt oder Nickel oder Legierungen dieser Materialien oder in Verbindung mit anderen Materialien eingesetzt werden, und nichtmagnetische Materialien mit magnetischen Materialien beschichtet eingesetzt werden.

5. Verfahren nach Anspruch 1, bei dem eine Mikrostruktur aus einem Polymer eingesetzt wird, welches magnetische Partikel enthält oder welches mit magnetischen Partikeln oder Materialien ganz oder teilweise beschichtet ist.

6. Verfahren nach Anspruch 1, bei dem die formschlüssige Verbindung der Mikrostruktur mit der immobilen Zelle durch die Form der Mikrostruktur realisiert wird.

7. Verfahren nach Anspruch 1, bei dem die Verbindung der Mikrostruktur mit der immobilen Zelle durch biochemische Funktionalisierung von Oberflächen realisiert wird.

8. Verfahren nach Anspruch 1, bei dem die Mikrostruktur mit Längen von 1 - 200 µm und Durchmessern von 1 µm bis 20 µm eingesetzt werden.

9. Verfahren nach Anspruch 1, bei dem ein sich zeitlich veränderndes dreidimensionales Magnetfeld durch Permanentmagneten oder Elektromagneten realisiert wird.

## Claims

1. In vitro method for mobilizing immobilized cells, in which an immobile cell is connected to a microstructure, the microstructure consisting at least partly of magnetic materials, and a non-reciprocal movement of the microstructure with the immobile cell being executed by means of a time-varying three-dimensional external magnetic field, immobile non-moving sperm cells being used as immobile cells, and a microstructure being used which has the shape of a helix structure or an artificial flexible flagellum.

2. Method according to Claim 1, in which a microstructure is used which is made of a magnetic material or of a material with magnetic particles.

3. Method according to Claim 1, in which ferromagnetic or paramagnetic material is used as magnetic material.

4. Method according to Claim 1, in which iron, iron oxide, cobalt or nickel or alloys of these materials or in conjunction with other materials is used as magnetic material, and non-magnetic materials coated with magnetic materials are used.

5. Method according to Claim 1, in which a microstructure is used which is made of a polymer which contains magnetic particles or which is wholly or partly coated with magnetic particles or materials.

6. Method according to Claim 1, in which the form-fitting connection of the microstructure to the immobile cell is implemented by the shape of the microstructure.

7. Method according to Claim 1, in which the connection of the microstructure to the immobile cell is implemented by biochemical functionalization of surfaces.

8. Method according to Claim 1, in which the microstructure used has lengths of 1 - 200 µm and diameters of 1 µm to 20 µm.

9. Method according to Claim 1, in which a time-varying three-dimensional magnetic field is implemented by permanent magnets or electromagnets.

## Revendications

1. Procédé in vitro de rétablissement de la mobilité de cellules immobiles, dans lequel on assemble une cellule immobile à une microstructure, dans lequel la microstructure se compose au moins en partie de matériaux magnétiques et on exécute un mouvement non réciproque de la microstructure avec la cellule immobile au moyen d'un champ magnétique externe tridimensionnel variable dans le temps, dans lequel on utilise comme cellules immobiles des spermatozoïdes stationnaires immobiles, et dans lequel on utilise une microstructure, qui présente la forme d'une structure hélicoïdale ou d'une flagelle artificielle flexible.

2. Procédé selon la revendication 1, dans lequel on utilise une microstructure en un matériau magnétique ou en un matériau avec des particules magnétiques.

3. Procédé selon la revendication 1, dans lequel on utilise comme matériau magnétique un matériau ferromagnétique ou paramagnétique.

4. Procédé selon la revendication 1, dans lequel on utilise comme matériau magnétique du fer, de l'oxyde de fer, du cobalt ou du nickel ou des alliages de ces matériaux ou en combinaison avec d'autres matériaux, et des matériaux non magnétiques revêtus avec des particules magnétiques.

5. Procédé selon la revendication 1, dans lequel on utilise une microstructure en un polymère, qui contient des particules magnétiques ou qui est revêtu en totalité ou en partie avec des particules ou des matériaux magnétiques.

6. Procédé selon la revendication 1, dans lequel on réalise l'assemblage par emboîtement de la microstructure avec la cellule immobile par la forme de la microstructure.

7. Procédé selon la revendication 1, dans lequel on réalise l'assemblage de la microstructure avec la cellule immobile par fonctionnalisation biochimique de surfaces.

8. Procédé selon la revendication 1, dans lequel on utilise la microstructure avec des longueurs de 1 - 200 µm et des diamètres de 1 µm à 20 µm.

9. Procédé selon la revendication 1, dans lequel on réalise un champ magnétique tridimensionnel variant dans le temps au moyen d'aimants permanents ou d'électroaimants.
